# EUROPEAN PATENT APPLICATION

(11) **EP 3 391 814 A1**
(43) Date of publication of application: **24.10.2018**
(21) Application number: 18167309.6
(22) Date of filing: 13.04.2018
(51) Int. Cl.: A61B 5/0428, A61B 5/042, A61B 5/0408, A61B 5/00

(54) **REFERENCE METHODOLOGY FOR ECG VECTOR RECONSTRUCTION**

(30) Priority: 19.04.2017 US 201715491891
(71) Applicant: Siemens Healthcare GmbH, 91052 Erlangen (DE)
(72) Inventor: KOERTGE, Detlef W., Carpentersville, Illinois 60110 (US); SZUMANSKI, Thomas, McHenry, Illinois 60051 (US)
(74) Representative: Patentanwälte Bals & Vogel

(57) **Abstract**

Embodiments provide a method and system for using a reconstructed or virtual reference point for electrocardiogram (ECG) measurements, allowing for the removal of the common reference point required in conventional ECG systems. Embodiments allow for either referencing measurements back to a reference previously digitized and reconstructed in the analog domain or to compare the measurements to a virtual reference. These calculations can be performed at the measurement site or remotely. Vector calculations can be made in either the analog or digital domains. A secondary means of measuring common mode noise (or rejection) injected into the ECG may be provided to ensure proper functioning in the presence of large common mode noise. These secondary measurements may be used to phase-align segments of the ECG vectors or as means to improve the common mode noise capabilities.

## Description

### BACKGROUND

Conventional electrocardiogram (ECG) systems require a common reference point (such as a Wilson Central Terminal (WCT)) to properly calculate ECG vectors. This common reference point on ECG and intra-cardiac ECG (IECG) requires a hard-wired reference connection between measurement points and measurement equipment. The hard-wired reference connection can be inconvenient and bothersome to both medical personnel and patients.

### SUMMARY

Embodiments of the present invention provide a method and system for using a reconstructed or virtual reference point for electrocardiogram (ECG) measurements, allowing for the removal of the common reference point required in conventional ECG systems.

In an embodiment, a method for electrocardiogram (ECG) vector reconstruction is provided, the method comprising: obtaining ECG measurements at a plurality of points on a patient with respective electrodes; calculating, by a processor in communication with the electrodes, a central terminal (CT) value based on the ECG measurements; and transmitting, by the processor, the CT value to a second device to be utilized as a reference point by the second device.

In an embodiment, the second device comprises a site electrode at a site of the patient, wherein the site electrode is connected to a monitoring device. The site electrode may be connected via a second wire or wirelessly to a monitoring device.

In an embodiment, the CT value creates a closed feedback loop.

In an embodiment, the transmitting is done wirelessly.

According to an embodiment, calculating the CT value comprises sampling the ECG measurements by an analog to digital converter to calculate the CT value in a digital domain. The second device may recreate the CT value in an analog domain for the second device to use as the reference point.

In an embodiment, the method for ECG vector reconstruction further comprises: obtaining, by the processor, secondary measurements relating to common mode noise of the ECG measurements; and utilizing, by the processor, the secondary measurements to phase-align segments of the reference point. In an embodiment, the secondary measurements are obtained by the processor through one or more of a low frequency radio-frequency (RF) antenna, direct electrical measurement of noise at the ECG measurement sites, and a separate device to measure phase of possible noise generators.

In an additional embodiment, a system for electrocardiogram (ECG) vector reconstruction is provided. The system comprises a processor configured to: receive, from a plurality of electrodes, ECG measurements at respective points on a patient; calculate a central terminal (CT) value based on the ECG measurements; and transmit the CT value to a second device to be utilized as a reference point by the second device.

### BRIEF DESCRIPTION OF THE DRAWINGS

The foregoing and other aspects of the present invention are best understood from the following detailed description when read in connection with the accompanying drawings. For the purpose of illustrating the invention, there is shown in the drawings embodiments that are presently preferred, it being understood, however, that the invention is not limited to the specific instrumentalities disclosed. Included in the drawings are the following Figures:
FIG. 1 is a diagram providing an example of using a reconstructed or virtual reference point for ECG measurements, according to embodiments;
FIG. 2 is a diagram providing another example of using a reconstructed or virtual reference point for ECG measurements, according to embodiments;
FIG. 3 is a diagram illustrating use of a reconstructed or virtual reference point for ECG measurements applied to electrophysiology applications, according to an embodiment;
FIG. 4 is a diagram illustrating use of a reconstructed or virtual reference point for ECG measurements for joining two surface electrodes not connected, according to an embodiment;
FIG. 5 is a concept diagram illustrating applications for use of reconstructed or virtual reference point for ECG measurements, according to an embodiment;
FIG. 6 is a flow chart illustrating a method of using a reconstructed or virtual reference point for ECG measurements, according to an embodiment; and
FIG. 7 is an exemplary computing environment in which embodiments disclosed herein may be implemented.

### DETAILED DESCRIPTION

Embodiments of the present invention relate to a method and system for using a reconstructed or virtual reference point for electrocardiogram (ECG) measurements. The virtual reference point conveniently allows for the removal of the common reference point required in conventional ECG systems.

Embodiments described herein allow for either referencing measurements back to a reference previously digitized and reconstructed in the analog domain or to compare the measurements to a virtual reference. These calculations can be performed at the measurement site or remotely. Vector calculations can be made in either the analog or digital domains.

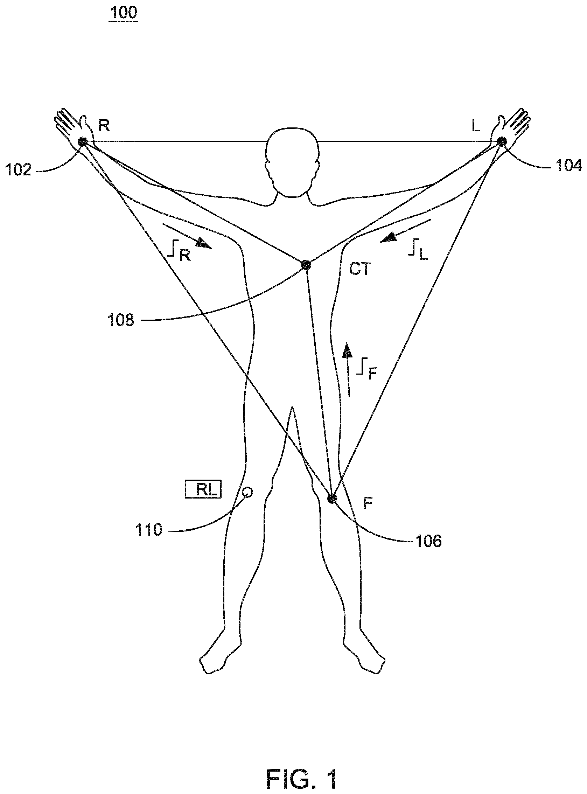

With reference to FIG. 1, a diagram 100 provides an example of using a reconstructed or virtual reference point for ECG measurements. Measurements at lead R (right arm) 102, L (left arm) 104, and F (foot or left leg) 106 are, according to an embodiment, averaged to determine the physiological value CT (central terminal) 108. The central terminal is of physiological significance for a number of reasons. One use of the CT 108 value is the RL (right leg) signal 110. CT 108 may be amplified, inverted, and driven back to RL 110 to create a closed feedback loop to eliminate common mode noise from the ECG. Reconstruction of CT 108 at RL 110, according to embodiments herein, allows for an electrode at RL 110 to be connected via a different mechanism than R 102, L 104, or F 106, such as through a second wire or wirelessly via telemetry, for example.

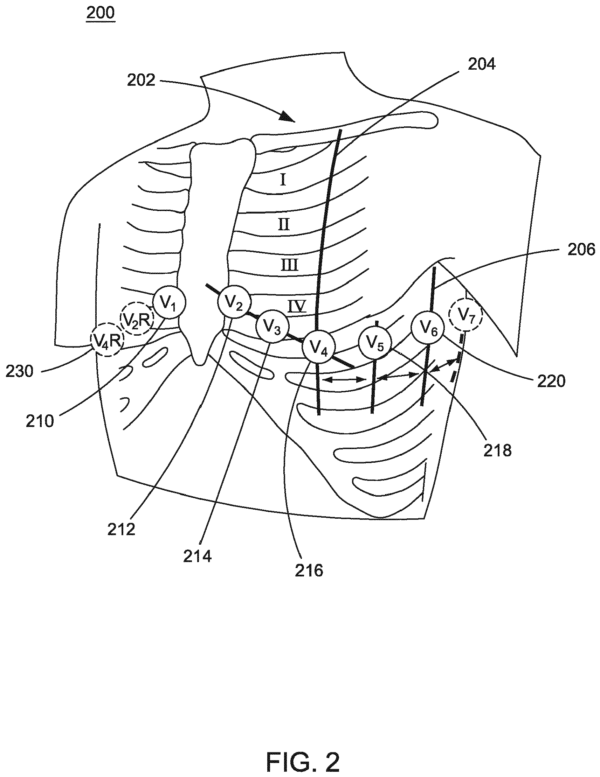

With reference to FIG. 2, a diagram 200 provides an additional example of using a reconstructed or virtual reference point for ECG measurements. Shown in FIG. 2 are clavicula 202, mid-clavicular line 204, and mid-axillary line 206. CT may be used as a reference to ECG precordial leads V1-V6 (210, 212, 214, 216, 218, and 220) as shown in FIG. 2. Monitoring of R, L, and F, as described with reference to FIG. 1, and sending CT to the V4R site 230 via telemetry allows for monitoring of the precordial lead sites without having a shared wired connection. The reference is sent back to the electrode site of V1-V6 (210, 212, 214, 216, 218, and 220). This is beneficial as wired connections to lead sites V1 to V6 in the angiography lab have been found to interfere with image quality as the wires will appear on the X-ray images.

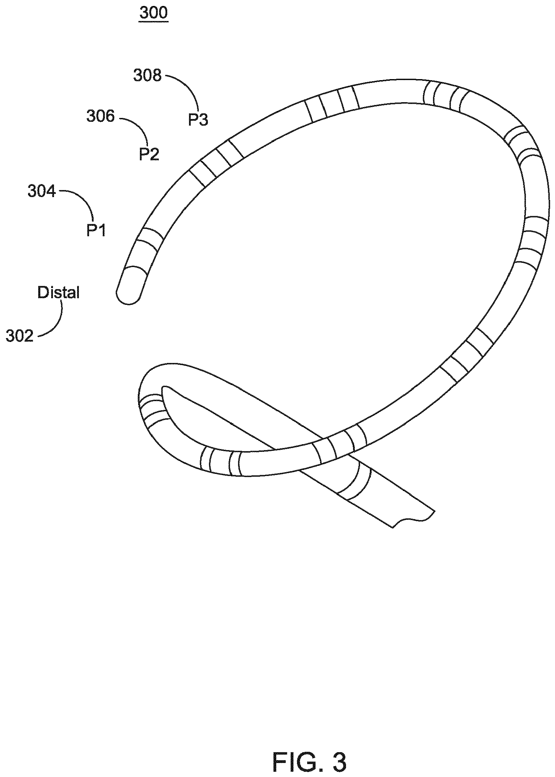

In yet another example, with reference to FIG. 3, a reconstructed or virtual reference point for ECG measurements may be applied to electrophysiology applications. A catheter 300 may be inserted into the heart to invasively measure electrical conduction pathways. The CT signal, according to embodiments herein, is used as a reference for these catheter connections. An example of this vector calculation is a measurement at distal electrode 302 subtracted from CT. This vector calculation is conventionally predicated on the catheter 300 and surface ECG connections P1-P3 (304, 306, and 308) sharing a common reference, requiring a wired connection between the two points. If CT is reconstructed in the circuitry monitoring the catheter connections, there is no need for a physical wired connection between the two points.

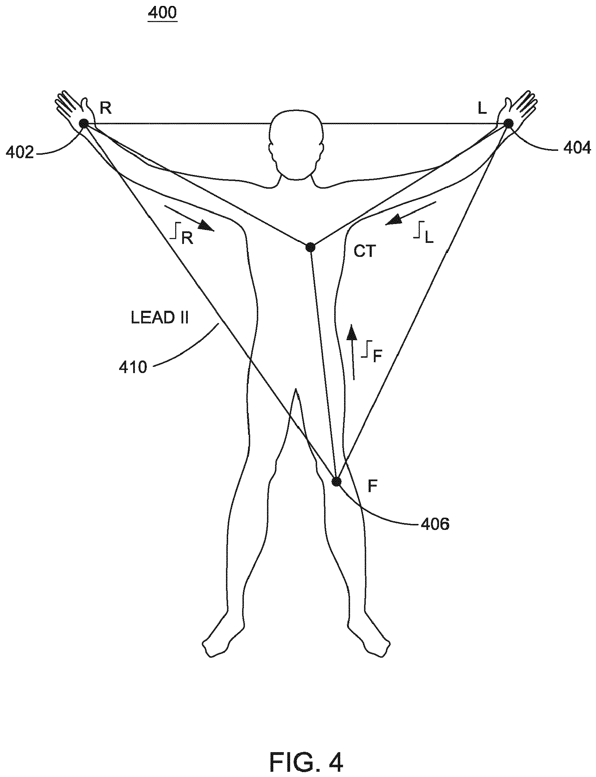

With reference to diagram 400 of FIG. 4, the reconstructed or virtual reference point for ECG measurements, according to embodiments herein, may be used to join two surface electrodes not connected via any other means. If R 402, L 404, and F 406 are floating with respect to one another, a wireless telemetry system may be created using an arbitrary reference locally at each site 402, 404, and 406. The vector calculation is: (F - Arbitrary Reference) - (R - Arbitrary Reference) = (F - R), allowing for the correct vector (F-R) = Lead II 410 to be created. Thus, two point sources may allow for a wireless reconstruction of Lead II 410 via telemetry at a remote patient monitor.

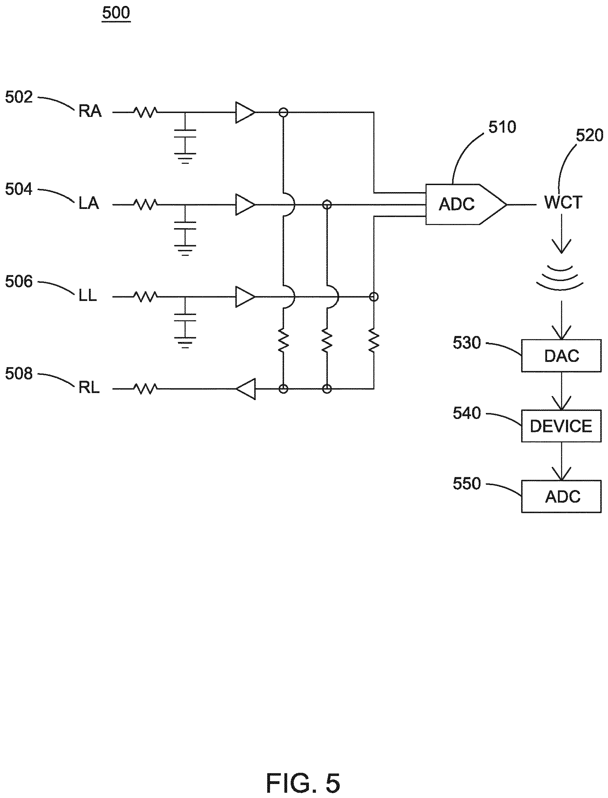

With reference to FIG. 5, a concept diagram 500 illustrates possible applications for use of reconstructed or virtual reference point for ECG measurements, according to an embodiment. RA 502, LA 504, and LL 506 are sampled by an analog to digital converter (ADC) 510. The ADC 510 calculates the WCT 520 in the digital domain. In an embodiment, the value of the WCT 520 is sent wirelessly to a digital to analog converter (DAC) 530. The DAC 530 recreates the WCT in the analog domain for a second device 540 to use as a reference.

The second device 540, according to an embodiment, uses the reference as part of its ECG vector reconstruction and is sampled by another ADC 550. The second device 540 may be the V-leads that require the WCT as a reference or a uni-polar catheter signal, for example. Similarly, RA 502 and LA 504 may be split using an arbitrary but fixed reference.

A secondary means of measuring common mode noise (or rejection) injected into the ECG may be needed to ensure proper functioning in the presence of large common mode noise. According to another embodiment, this secondary means may include, but is not limited to, a low frequency radio-frequency (RF) antenna, direct electrical measurement of noise at the ECG measurement site, or a separate device to measure phase of possible noise generators at the source. These secondary measurements may be used to phase-align segments of the ECG vectors or as means to improve the common mode noise capabilities.

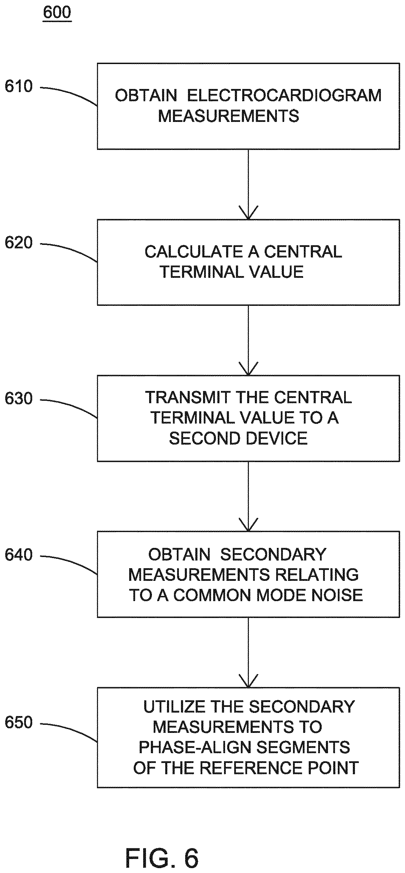

With reference to the flowchart 600 of FIG. 6, method of using a reconstructed or virtual reference point for ECG measurements, according to an embodiment, is illustrated. At 610, ECG measurements at a plurality of points on a patient with respective electrodes are obtained. At 620, a CT value based on the ECG measurements is calculated. At 630, the CT value is transmitted to a second device to be utilized as a reference point by the second device.

In an additional embodiment, at 640, secondary measurements relating to common mode noise of the ECG measurements are obtained. At 650, the secondary measurements are utilized to phase-align segments of the reference point.

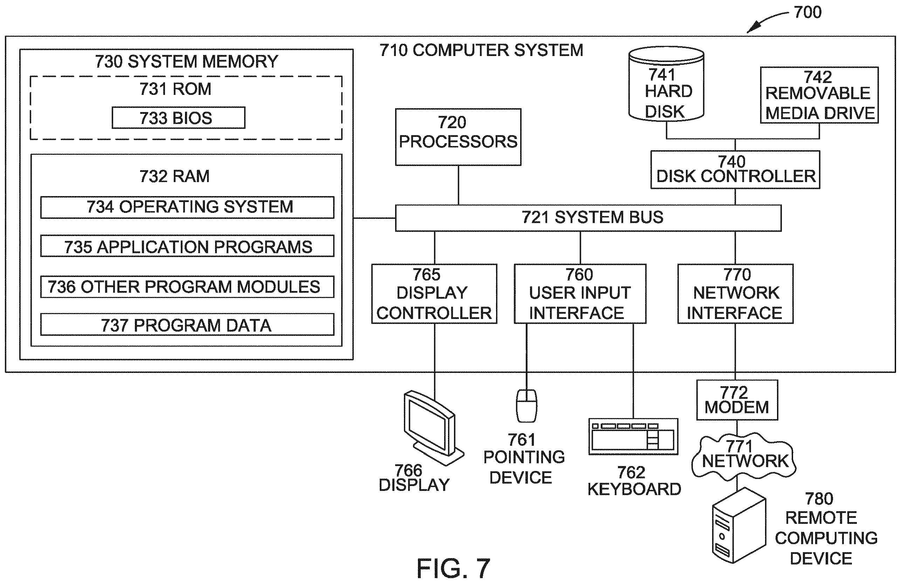

FIG. 7 illustrates an exemplary computing environment 700 within which embodiments of the invention may be implemented. Computing environment 700 may include computer system 710, which is one example of a computing system upon which embodiments of the invention may be implemented. Computers and computing environments, such as computer 710 and computing environment 700, are known to those of skill in the art and thus are described briefly here.

As shown in FIG. 7, the computer system 710 may include a communication mechanism such as a bus 721 or other communication mechanism for communicating information within the computer system 710. The system 710 further includes one or more processors 720 coupled with the bus 721 for processing the information. The processors 720 may include one or more central processing units (CPUs), graphical processing units (GPUs), or any other processor known in the art.

The computer system 710 also includes a system memory 730 coupled to the bus 721 for storing information and instructions to be executed by processors 720. The system memory 730 may include computer readable storage media in the form of volatile and/or nonvolatile memory, such as read only memory (ROM) 731 and/or random access memory (RAM) 732. The system memory RAM 732 may include other dynamic storage device(s) (e.g., dynamic RAM, static RAM, and synchronous DRAM). The system memory ROM 731 may include other static storage device(s) (e.g., programmable ROM, erasable PROM, and electrically erasable PROM). In addition, the system memory 730 may be used for storing temporary variables or other intermediate information during the execution of instructions by the processors 720. A basic input/output system 733 (BIOS) containing the basic routines that help to transfer information between elements within computer system 710, such as during start-up, may be stored in ROM 731. RAM 732 may contain data and/or program modules that are immediately accessible to and/or presently being operated on by the processors 720. System memory 730 may additionally include, for example, operating system 734, application programs 735, other program modules 736 and program data 737.

The computer system 710 also includes a disk controller 740 coupled to the bus 721 to control one or more storage devices for storing information and instructions, such as a magnetic hard disk 741 and a removable media drive 742 (e.g., floppy disk drive, compact disc drive, tape drive, and/or solid state drive). The storage devices may be added to the computer system 710 using an appropriate device interface (e.g., a small computer system interface (SCSI), integrated device electronics (IDE), Universal Serial Bus (USB), or FireWire).

The computer system 710 may also include a display controller 765 coupled to the bus 721 to control a display or monitor 766, such as a cathode ray tube (CRT) or liquid crystal display (LCD), for displaying information to a computer user. The computer system 710 includes an input interface 760 and one or more input devices, such as a keyboard 762 and a pointing device 761, for interacting with a computer user and providing information to the processors 720. The pointing device 761, for example, may be a mouse, a trackball, or a pointing stick for communicating direction information and command selections to the processors 720 and for controlling cursor movement on the display 766. The display 766 may provide a touch screen interface which allows input to supplement or replace the communication of direction information and command selections by the pointing device 761.

The computer system 710 may perform a portion or all of the processing steps of embodiments of the invention in response to the processors 720 executing one or more sequences of one or more instructions contained in a memory, such as the system memory 730. Such instructions may be read into the system memory 730 from another computer readable medium, such as a hard disk 741 or a removable media drive 742. The hard disk 741 may contain one or more datastores and data files used by embodiments of the present invention. Datastore contents and data files may be encrypted to improve security. The processors 720 may also be employed in a multi-processing arrangement to execute the one or more sequences of instructions contained in system memory 730. In alternative embodiments, hard-wired circuitry may be used in place of or in combination with software instructions. Thus, embodiments are not limited to any specific combination of hardware circuitry and software.

As stated above, the computer system 710 may include at least one computer readable medium or memory for holding instructions programmed according to embodiments provided herein and for containing data structures, tables, records, or other data described herein. The term "computer readable medium" as used herein refers to any medium that participates in providing instructions to the processors 720 for execution. A computer readable medium may take many forms including, but not limited to, non-volatile media, volatile media, and transmission media. Non-limiting examples of non-volatile media include optical disks, solid state drives, magnetic disks, and magneto-optical disks, such as hard disk 741 or removable media drive 742. Non-limiting examples of volatile media include dynamic memory, such as system memory 730. Non-limiting examples of transmission media include coaxial cables, copper wire, and fiber optics, including the wires that make up the bus 721. Transmission media may also take the form of acoustic or light waves, such as those generated during radio wave and infrared data communications.

The computing environment 700 may further include the computer system 710 operating in a networked environment using logical connections to one or more remote computers, such as remote computer 780. Remote computer 780 may be a personal computer (laptop or desktop), a mobile device, a server, a router, a network PC, a peer device or other common network node, and typically includes many or all of the elements described above relative to computer system 710. When used in a networking environment, computer system 710 may include modem 772 for establishing communications over a network 771, such as the Internet. Modem 772 may be connected to system bus 721 via user network interface 770, or via another appropriate mechanism.

Network 771 may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 710 and other computers (e.g., remote computing system 780). The network 771 may be wired, wireless or a combination thereof. Wired connections may be implemented using Ethernet, Universal Serial Bus (USB), RJ-11 or any other wired connection generally known in the art. Wireless connections may be implemented using Wi-Fi, WiMAX, and Bluetooth, infrared, cellular networks, satellite or any other wireless connection methodology generally known in the art. Additionally, several networks may work alone or in communication with each other to facilitate communication in the network 771.

As described herein, the various systems, subsystems, agents, managers and processes can be implemented using hardware components, software components and/or combinations thereof.

Although the present invention has been described with reference to exemplary embodiments, it is not limited thereto. Those skilled in the art will appreciate that numerous changes and modifications may be made to the preferred embodiments of the invention and that such changes and modifications may be made without departing from the true spirit of the invention. It is therefore intended that the appended claims be construed to cover all such equivalent variations as fall within the true spirit and scope of the invention.

## Claims

1. A method for electrocardiogram (ECG) vector reconstruction, the method comprising:
obtaining ECG measurements at a plurality of points on a patient with respective electrodes;
calculating, by a processor in communication with the electrodes, a central terminal (CT) value based on the ECG measurements; and
transmitting, by the processor, the CT value to a second device to be utilized as a reference point by the second device.

2. The method according to claim 1, wherein the second device comprises a site electrode at a site of the patient, wherein the site electrode is connected to a monitoring device.

3. The method according to claim 2, wherein the site electrode is connected via a second wire or wirelessly to a monitoring device.

4. The method according to any of the preceding claims, wherein the CT value creates a closed feedback loop.

5. The method according to any of the preceding claims, wherein the transmitting is done wirelessly.

6. The method of the preceding claims, wherein calculating the CT value comprises sampling the ECG measurements by an analog to digital converter to calculate the CT value in a digital domain.

7. The method according to claim 6, wherein the second device recreates the CT value in an analog domain for the second device to use as the reference point.

8. The method according to any of the preceding claims, further comprising:
obtaining, by the processor, secondary measurements relating to common mode noise of the ECG measurements; and
utilizing, by the processor, the secondary measurements to phase-align segments of the reference point.

9. The method according to of claim 8, wherein the secondary measurements are obtained by the processor through one or more of a low frequency radio-frequency (RF) antenna, direct electrical measurement of noise at the ECG measurement sites, and a separate device to measure phase of possible noise generators.

10. A system for electrocardiogram (ECG) vector reconstruction, the system comprising:
a processor configured to:
receive, from a plurality of electrodes, ECG measurements at respective points on a patient;
calculate a central terminal (CT) value based on the ECG measurements; and
transmit the CT value to a second device to be utilized as a reference point by the second device.

11. The system according to claim 10, wherein the second device comprises a site electrode at a site of the patient, wherein the site electrode is connected to a monitoring device.

12. The system according to claim 11, wherein the site electrode is connected via a second wire or wirelessly to a monitoring device.

13. The system according to any of the preceding claims 10 to 12, wherein the CT value creates a closed feedback loop.

14. The system according to any of the preceding claims 10 to 13, wherein the transmitting is done wirelessly.

15. The system according to any of the preceding claims 10 to 14, wherein calculating the CT value comprises sampling the ECG measurements by an analog to digital converter to calculate the CT value in a digital domain.

16. The system according to claim 15, wherein the second device recreates the CT value in an analog domain for the second device to use as the reference point.

17. The system according to any of the preceding claims 10 to 16, wherein the processor is further configured to:
obtain secondary measurements relating to common mode noise of the ECG measurements; and
utilize the secondary measurements to phase-align segments of the reference point.

18. The system according to claims 17, wherein the secondary measurements are obtained through one or more of a low frequency radio-frequency (RF) antenna, direct electrical measurement of noise at the ECG measurement sites, and a separate device to measure phase of possible noise generators.
